# EUROPEAN PATENT SPECIFICATION

(11) **EP 0 516 703 B1**
(45) Date of publication and mention of the grant of the patent: **07.06.1995**
(21) Application number: 91904979.1
(22) Date of filing: 20.02.1991
(51) Int. Cl.: F21V 23/04, A43B 1/00

(54) **SHOE INCORPORATING PIEZOELECTRIC MATERIAL**
SCHUH MIT PIEZO-ELEKTRISCHEM MATERIAL
CHAUSSURE AVEC MATERIAU PIEZOELECTRIQUE INTEGRE

(30) Priority: 20.02.1990 GB 9003810; 24.05.1990 GB 9011681
(43) Date of publication of application: 09.12.1992
(73) Proprietor: Mott, Jonathan Christopher, Bentley, Farnham, Surrey GU10 5EU (GB)
(72) Inventor: Mott, Jonathan Christopher, Bentley, Farnham, Surrey GU10 5EU (GB)
(74) Representative: Shackleton, Nicola
(86) International application number: GB9100267
(87) International publication number: WO9113288

(56) References cited:
- WO-A-87/02846
- NL-A- 8 006 456
- US-A- 3 549 878
- US-A- 3 610 916
- US-A- 3 828 177
- US-A- 4 128 861
- PATENT ABSTRACTS OF JAPAN vol. 8, no. 42 (P-256)(1479) 23 February 1984, & JP-A- 58 195238

## Description

This invention relates to a shoe incorporating piezoelectric material.

It has already been proposed, for example, in U.S Patent No. 4,748,366, to provide dancing shoes and a child's ball each incorporating a layer of piezoelectric material and a light source. The piezoelectrical material and the light source are directly electrically connected together so that the electrical energy produced by piezoelectric effect upon impact of the shoe or ball against, e.g the ground, directly energises the light source.

However, the amount of electrical energy normally produced in response to a typical impact is insufficient to produce readily visible energisation of the light source, i.e., energisation of the light source which is clearly visible in daylight and/or of significant duration. It is, therefore, an object of the present invention to alleviate this problem.

Related prior art can be found in U.S. Patent No. 3,610,916 which discloses an illuminable ball with an inertia switch and a time delay circuit. The ball is translucent and within the ball is an inertia switch which triggers a time-delay circuit that then applies battery power to two lamps. Power is applied for a duration preselected by the time-delay circuit.

In addition, U.S. Patent No. 4,128,861 discloses an illuminated shoe having a lamp and battery circuit disposed within the heel of the shoe and a means for activating the lamp comprising contacts on the heel of the shoe. Upon application of pressure to the heel, the contacts are pressed together causing the battery circuit to close and the lamp to light.

According to the present invention, there is provided a shoe having a sole, said shoe adapted to emit light or sound energy in response to the impact of said sole on a surface, said sole comprising a battery-powered light-emitting or sound-emitting unit, said unit comprising an impact-sensing element, a battery, a light-emitting or sound-emitting device, characterised in that said impact-sensing element is made from polymeric piezoelectric material and in that said unit further comprises a circuit connected to said piezoelectric material, said circuit being triggered by electrical energy from said piezoelectric material in response to the impact of the sole on a surface thereby energising said light-emitting or sound-emitting device from said battery in response to the electrical energy produced by the impact.

Thus, the electrical energy resulting from each impact can be used merely as a trigger to operate the light-emitting or sound emitting unit via the circuit incorporated therein, and the amount and/or duration of the light or sound emission can be independently determined/controlled by appropriate design of the circuit.

The circuit preferably comprises a monostable circuit whose time constant determines the duration of energisation of the light-emitting or sound-emitting device and may advantageously be adjustable, for example by means of a variable resistance.

The light-emitting device, may be a light-emitting diode or a gas discharge lamp such as a neon lamp.

Preferably, the shoe has a sole in the form of a moulded part, preferably a unitary sole-and-heel structure, with at least the battery, the circuit and a light-emitting device disposed in or moulded into the heel part of the structure, and with the light-emitting device being arranged to emit light rearwardly from said heel part. However, the polymeric piezoelectric material may be moulded into the sole part of the structure, preferably in the region of maximum stress.

Another embodiment of the shoe may include a plurality of light-emitting devices, either positioned at a plurality of points over the outside upper surface of the shoe or connected to said plurality of points via respective optical fibres, said devices being selectively energisable in a predetermined sequence, for example in response to each successive group of a predetermined number of impacts sensed by the impact-sensing element. This embodiment is particularly suitable for use as an exercise shoe in performing sequences of different exercises, since different light-emitting devices, perhaps differently coloured, can be energised for each different exercise.

Alternatively, the shoe with a plurality of light-emitting devices can also incorporate a plurality of impact-sensing elements distributed throughout the sole of the shoe, the circuit being arranged to energise the light-emitting devices in dependence upon the relative stress sensed by each impact-sensing element so as to provide an indication of the weight distribution over the sole of the shoe. This embodiment is particularly suitable for use as an athletic, gymnastic or dancing training shoe, since it can provide useful information on the style or angle of foot-ground contact of an athlete, gymnast or dancer wearing it.

The optical fibre means described may comprise a single optical fibre at least a part of which runs around said outside upper surface of the shoe, said part having a plurality of notches therein so as to emit light at each notch. The notches may be coloured, e.g., with different coloured translucent inks or dyes, so that the emitted light is correspondingly coloured.

Alternatively, the optical fibre means may comprise a bundle of optical fibres, each fibre conducting said emitted light to a different point in the outside upper surface of the shoe: for example, where the shoe has a tongue, at least some of the fibres may terminate in the free end of the tongue. Said points may form an abstract or geometrical pattern, or a logo or one or more alphanumeric characters constituting, for example, a trade mark of the manufacturer of the shoe. The ends of the fibres at said points may be coloured, e.g., with different coloured translucent inks or dyes.

The invention will now be described, by way of non-limitative example only, with reference to the accompanying drawings, of which:
Figure 1 shows, somewhat schematically, a running- or jogging-shoe in accordance with the present invention;
Figure 2 shows a simplified circuit for incorporation into the shoe of Figure 1;
Figures 3 to 7 show further embodiments of shoes in accordance with the present invention.

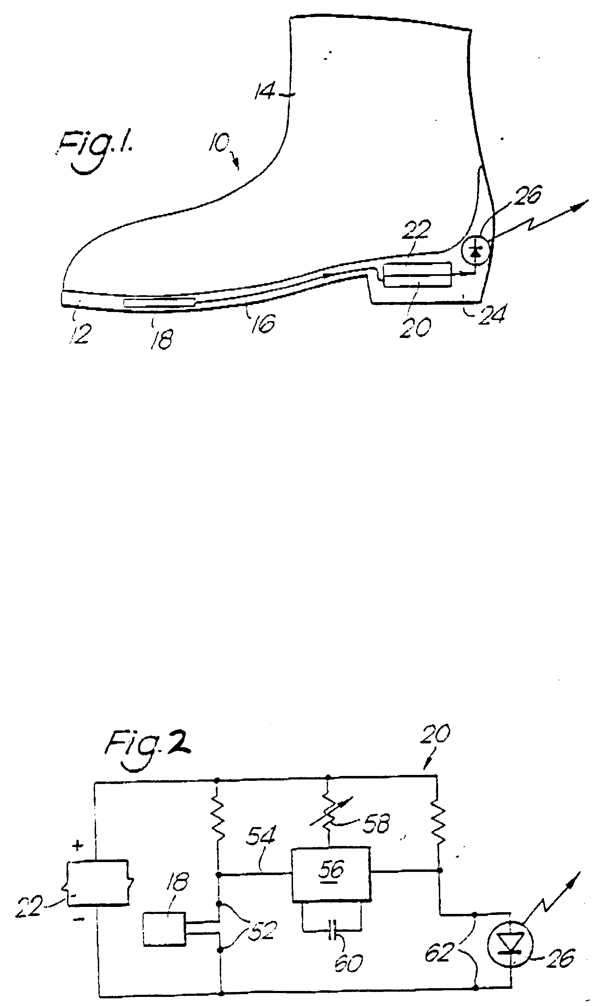

The shoe of Figure 1 is indicated generally at 10, and comprises a unitary sole-and-heel structure 12 moulded to an upper 14. The moulded sole-and-heel structure 12 may be moulded using a method generally along the lines of that described in my United Kingdom Patent Application No. 8915923 (Publication No. 2 235 150), filed on 12th July 1989.

Moulded within the sole part 16 of the sole-and-heel structure 12, adjacent the point of maximum stress (i.e. near the part corresponding to the ball of the wearer's foot) is a thin rectangular sheet or layer 18 of polymeric piezoelectric material, preferably of polyvinylidene fluoride (PVDF), which has been stretch oriented and electrically polarised to enhance its piezoelectric properties. The sheet 18 is electrically connected to a battery-powered circuit 20 which together with its lithium battery pack 22 is moulded into the heel part 24 of the sole-and-heel structure 12. The circuit 20, which basically comprises a monostable circuit and will be described in more detail hereinafter, is connected to energise a light-emitting diode (LED) 26, which is moulded into the heel part 24 so as to face rearwardly therefrom.

In use, as the wearer of the shoe 10 runs or jogs, the layer 18 of piezoelectric material produces a pulse of electrical energy each time the sole part 16 of the structure 12 impacts the ground, i.e., at each step or stride of the wearer, by virtue of the piezoelectric effect. Each pulse of electrical energy triggers the monostable circuit within the circuit 20, which in turn energises the light-emitting diode 26 from the battery pack 22. A bright flash of light is therefore emitted rearwardly from the heel of the shoe 10. Thus the wearer of a pair of the shoes 10 is clearly visible from behind by virtue of the flashes of light emitted rearwardly with each stride, which has considerable advantages from a safety point of view for runners and joggers on public roads, particularly in low light conditions.

Figure 2 shows the circuit 20 in Figure 1 in a little more detail. Thus the circuit 20 comprises a pair of inputs 52 connected externally to the sheet 18 of PVDF, and internally to the control input 54 of a monostable circuit 56. The monostable circuit 56 has a timing circuit constituted by a resistor 58 and a capacitor 60, power supply inputs connected to the battery pack 22, and outputs 62 connected to the LED 26. Adjustment or selection of the value of the resistor 58 determines the duration of the time period for which the monostable circuit 56 energises the LED 26 in response to each impact to which sheet 18 of PVDF is subjected.

Many modifications can be made to the described embodiments of the invention. In particular, the PVDF sheet 18, the circuit 20, the battery pack 22 and the LED 26 of the shoe 10 of Figure 1 can be formed as a single encapsulated unit which fits removably into the heel part 24 of the shoe. The LED 26 of this unit is arranged to align with an optical fibre bundle or other light guiding medium (e.g., a layer of suitable translucent plastic material) moulded into the heel part 24, so as to guide the light emitted by the LED to an external surface of the shoe, e.g., to a point exit facing rearwardly from the heel part 24 as before, or to one or more ribs or spines extending vertically up and down the heel part. This enables the encapsulated unit simply to be replaced when the battery pack 22 is exhausted, or when it is desired to change a unit with an LED of one colour for a unit with an LED of another colour. Alternatively, just the battery pack 22 can be arranged to be replaceable.

Additionally, the LEDs 26 can, if desired, be replaced by sound-emitting devices and the output of the circuit 20 in the shoe 10 can be arranged to operate other devices, e.g, an electronic pedometer.

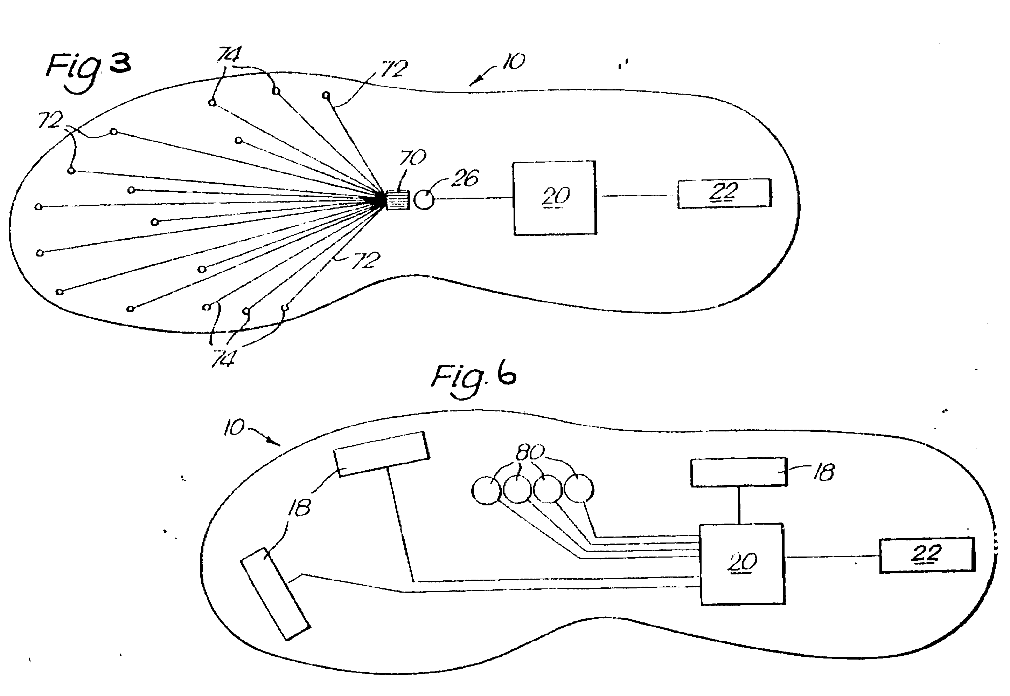

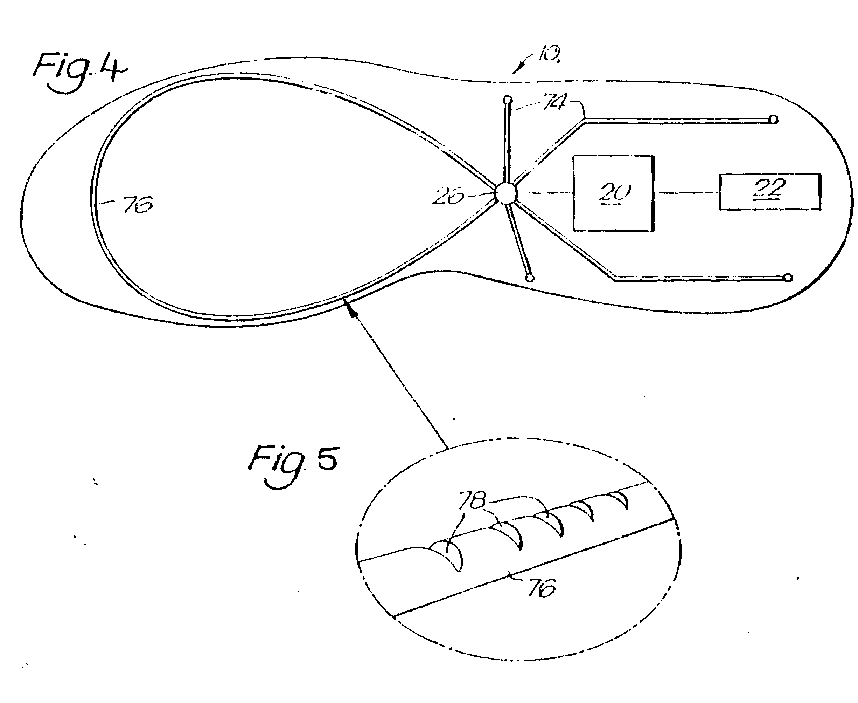

In embodiments of the shoe 10 where the LED 26 is used in conjunction with an optical fibre bundle, the individual fibres can be arranged to conduct the light from the LED to respective points on the outside surface of the shoe, e.g., distributed in an abstract or a geometric pattern over the upper part of the front of the shoe, or distributed to form a logo or one or more alphanumeric characters constituting a trade mark of the manufacturer of the shoe, or disposed throughout the free end of the tongue of the shoe: such an embodiment is illustrated in Figure 3 with the optical fibre bundle, the individual fibres and the points on the outside surface of the shoe being indicated at 70,72,74 respectively. Alternatively, and as shown in Figures 4 and 5, at least a part of the optical fibre bundle can be replaced by a single optical fibre 76 which runs around the upper part of the front of the shoe and which has a plurality of notches 78 distributed along its length from each of which light from the LED 26 is emitted. These notches 78, as well as the ends 74 of the individual fibres 72 of the optical fibre bundle 70 can be coloured, for example, with different coloured translucent inks or dyes, to create a variety of multi-coloured effects.

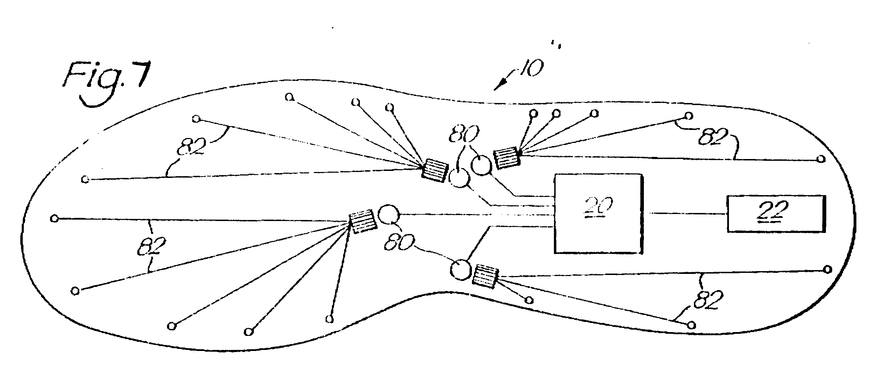

The shoe 10 can also be provided with a plurality of LEDs like the LED 26, either distributed over the outside upper surface of the shoe as shown at 80 in Figure 6 or disposed within the shoe as shown at 82 in Figure 7 and coupled to a plurality of points in the outside upper surface of the shoe via respective optical fibres 82. In this case, the circuit 20 can include a counter and gating circuit arranged to count successive impacts sensed by a single PVDF sheet 18 and to sequentially energise successive ones of the LEDs, say ten or twenty times each. This arrangement is particularly useful in an exercise shoe, since it can be used to assist and/or indicate the timing of a sequence of different exercises, with perhaps a different colour emitted for each different exercise.

In addition to a plurality of LEDs, the shoe 10 can also incorporate a plurality of individual PVDF sheets 18 distributed throughout the sole of the shoe, as also shown in Figure 6. In this case, the circuit 20 is arranged to energise respective ones of the LEDs in dependence upon the relative stress sensed by respective ones of the PVDF sheets, so as to provide an indication of the weight distribution over the sole of the shoe. This forms an excellent training aid for athletes, particularly runners, as well as for gymnasts and dancers, more especially if the athlete, gymnast or dancer is filmed in action with a video camera and the film is then played back in slow motion.

In embodiments of the shoe 10 involving optical fibre bundles, the LEDs 26,42 can, if desired, be omitted altogether, their effect being achieved simply by use of ambient light. Thus the fibre bundles act as light gatherers, so if their input ends are disposed say all over the front of the shoe facing generally forwardly and upwardly, while their output ends are disposed in the heel, facing rearwardly and distributed to form, for example, a logo or alphanumeric character consituting a trade mark as mentioned earlier, ambient light will in many circumstances be sufficient to make the output ends of the fibre appear to light up. Again, multicoloured effects can be created if desired, as described earlier.

## Claims

1. A shoe having a sole (16), said shoe adapted to emit light or sound energy in response to the impact of said sole (16) on a surface, said sole (16) comprising a battery-powered light-emitting or sound-emitting unit, said unit comprising an impact-sensing element (18), a battery (22), a light-emitting or sound-emitting device (26,34), characterised in that said impact-sensing element (18) is made from polymeric piezoelectric material and in that said unit further comprises a circuit (20) connected to said piezoelectric material (18), said circuit (20) being triggered by electrical energy from said piezoelectric material (18) in response to the impact of the sole (16) on a surface thereby energising said light-emitting or sound-emitting device (26,34) from said battery (22) in response to the electrical energy produced by the impact.

2. A shoe as claimed in Claim 1, characterised in that circuit (20) comprises a monostable circuit (56) whose time constant determines the duration of energisation of the light-emitting or sound-emitting device.

3. A shoe as claimed in Claim 2, including means (58,60,62) for adjusting the time constant of the monostable circuit (56).

4. A shoe as claimed in Claim 3, characterised in that the adjusting means (58,60,62) comprises a variable resistance (58).

5. A shoe as claimed in any preceding claim, including a light-emitting diode (26) as said light-emitting device (26).

6. A shoe as claimed in any preceding claim, comprising a light-emitting device (26) disposed inside the sole (16) and further comprising optical fibre means (70,72,74,76) arranged to conduct light emitted by said light-emitting device (26) to an outside surface of the sole (16).

7. A shoe as claimed in any of Claims 1 to 5, characterised in that said sole (16) is a sole-and-heel structure (12) moulded as a unitary part with at least the battery (22), the circuit (20) and a light-emitting device (26) disposed in the heel part of the structure (12).

8. A shoe as claimed in Claim 7, characterised in that said light-emitting device (26) is arranged to emit light rearwardly from said heel part (24).

9. A shoe as claimed in Claim 7 or Claim 8, characterised in that said polymeric piezoelectric material (18) is moulded into the sole part (16) of the structure (12).

10. A shoe as claimed in any of Claims 1 to 5, including a plurality of light-emitting devices (26) positioned at a plurality of points over the outside upper surface (80) of the shoe.

11. A shoe as claimed in any of Claims 1 to 5, including a plurality of light-emitting devices (26) coupled to a plurality of points over the outside upper surface (80) of the shoe via respective optical fibres (82).

12. A shoe as claimed in Claim 10 or Claim 11, characterised in that said devices are energised in a predetermined sequence in response to successive impacts or groups of impacts sensed by the impact-sensing elements (18).

13. A shoe as claimed in Claim 10 or Claim 11, comprising a plurality of impact-sensing elements (18) distributed throughout the sole (16) of the shoe, the circuit (20) being arranged to energise the light-emitting devices (26) in dependence upon the relative stress sensed by each impact-sensing element (18) so as to provide an indication of the weight distribution over the sole (16) of the shoe.

14. A shoe as claimed in Claim 6, characterised in that the optical fibre means (70) comprises a single optical fibre (76) at least a part of which runs around the outside upper surface (80) of the shoe, said part having a plurality of notches (78) therein so as to emit light at each notch.

15. A shoe as claimed in Claim 14, characterised in that the notches (78) are coloured with coloured translucent inks or dyes, so that the emitted light is correspondingly coloured.

16. A shoe as claimed in Claim 6, wherein the optical fibre means (70) comprises a bundle of optical fibres (72,74) said fibres conducting said emitted light to a plurality of points (74) over the outside upper surface (80) of the shoe.

17. A shoe as claimed in Claim 16, said shoe including a tongue and at least some of the fibres (72,74) terminating in the free end of the tongue.

18. A shoe as claimed in any one of Claims 10, 11 and 16, characterised in that said points are arranged to form an abstract or geometric pattern, a logo or one or more alphanumeric characters.

19. A shoe as claimed in Claim 18, characterised in that the ends of the fibres at said points are coloured with coloured translucent inks or dyes.

## Patentansprüche

1. Schuh mit einer Sohle (16), wobei der Schuh dazu geeignet ist, Licht- oder Schallenergie in Antwort auf das Auftreffen der Sohle (16) auf eine Oberfläche zu emittieren, wobei die Sohle (16) eine batteriebetriebene lichtemittierende oder schallemittierende Einheit umfaßt, wobei die Einheit ein Element (18) zum Erfassen des Auftreffens umfaßt, eine Batterie (22), eine lichtemittierende oder schallemittierende Vorrichtung (26, 34),
dadurch gekennzeichnet,
daß das Element (18) zum Erfassen des Auftreffens aus polymerischem, piezoelektrischem Material hergestellt ist, und daß die Einheit ferner eine Schaltung (20) umfaßt, welche mit dem piezoelektrischen Material (18) verbunden ist, wobei die Schaltung (20) durch elektrische Energie von dem piezoelektrischen Material (18) in Antwort auf das Auftreffen der Sohle (16) auf eine Oberfläche getriggert wird, wodurch die lichtemittierende oder schallemittierende Vorrichtung (26, 34) durch die Batterie (22) in Antwort auf die durch das Auftreffen erzeugte elektrische Energie erregt wird.

2. Schuh nach Anspruch 1,
dadurch gekennzeichnet,
daß die Schaltung (20) eine monostabile Schaltung (56) umfaßt, deren Zeitkonstante die Dauer des Erregens der lichtemittierenden oder schallemittierenden Vorrichtung bestimmt.

3. Schuh nach Anspruch 2, umfassend ein Mittel (58, 60, 62) zum Einstellen der Zeitkonstante der monostabilen Schaltung (56).

4. Schuh nach Anspruch 3,
dadurch gekennzeichnet,
daß das Einstellmittel (58, 60, 62) einen veränderbaren Widerstand (58) umfaßt.

5. Schuh nach einem der vorhergehenden Ansprüche, umfassend eine lichtemittierende Diode (26) als die lichtemittierende Vorrichtung (26).

6. Schuh nach einem der vorhergehenden Ansprüche, umfassend eine lichtemittierende Vorrichtung (26), welche innerhalb der Sohle (16) angeordnet ist, und ferner umfassend ein optisches Fasermittel (70, 72, 74, 76), welches dazu eingerichtet ist, durch die lichtemittierende Vorrichtung (26) emittiertes Licht zu einer äußeren Oberfläche der Sohle (16) zu leiten.

7. Schuh nach einem der Ansprüche 1 bis 5,
dadurch gekennzeichnet,
daß die Sohle (16) ein als ein Teil geformter Sohlen-Absatz-Aufbau (12) ist, wobei wenigstens die Batterie (22), die Schaltung (20) und die lichtemittierende Vorrichtung (26) in dem Absatzteil des Aufbaus (22) angeordnet sind.

8. Schuh nach Anspruch 7,
dadurch gekennzeichnet,
daß die lichtemittierende Vorrichtung (26) dazu eingerichtet ist, Licht von dem Absatzteil (24) nach hinten zu emittieren.

9. Schuh nach Anspruch 7 oder Anspruch 8,
dadurch gekennzeichnet,
daß das polymerische, piezoelektrische Material (18) in den Sohlenteil (16) des Aufbaus (12) eingeformt ist.

10. Schuh nach einem der Ansprüche 1 bis 5, umfassend eine Mehrzahl lichtemittierender Vorrichtungen (26), welche an einer Mehrzahl von Punkten an der äußeren oberen Oberfläche (80) des Schuhs positioniert sind.

11. Schuh nach einem der Ansprüche 1 bis 5, umfassend eine Mehrzahl lichtemittierender Vorrichtungen (26), welche mit einer Mehrzahl von Punkten an der äußeren oberen Oberfläche (80) des Schuhs über jeweilige optische Fasern (82) gekoppelt sind.

12. Schuh nach Anspruch 10 oder Anspruch 11,
dadurch gekennzeichnet,
daß die Vorrichtungen in einer vorbestimmten Sequenz in Antwort auf aufeinanderfolgende Stöße oder Gruppen von Stößen, welche durch die Elemente (18) zum Erfassen des Auftreffens erfaßt werden, erregt werden.

13. Schuh nach Anspruch 10 oder Anspruch 11, umfassend eine Mehrzahl von Elementen (18) zum Erfassen des Auftreffens, welche über die Sohle (16) des Schuhs verteilt sind, wobei die Schaltung (20) dazu eingerichtet ist, die lichtemittierenden Vorrichtungen (26) in Abhängigkeit der relativen Belastung zu erregen, welche durch jedes Element (18) zum Erfassen des Auftreffens erfaßt wird, um eine Anzeige der Gewichtsverteilung auf der Sohle (16) des Schuhs vorzusehen.

14. Schuh nach Anspruch 6,
dadurch gekennzeichnet,
daß das optische Fasermittel (70) eine einzige optische Faser (76) umfaßt, von welcher wenigstens ein Teil um die äußere obere Oberfläche (80) des Schuhs herum verläuft, wobei der Teil eine Mehrzahl von Einkerbungen (78) darin aufweist, um bei jeder Einkerbung Licht zu emittieren.

15. Schuh nach Anspruch 14,
dadurch gekennzeichnet,
daß die Einkerbungen (78) mit durchscheinenden Farbstoffen oder Färbungen gefärbt sind, so daß das emittierte Licht entsprechend gefärbt ist.

16. Schuh nach Anspruch 6, worin das optische Fasermittel (70) ein Bündel optischer Fasern (72, 74) umfaßt, wobei die Fasern das emittierte Licht zu einer Mehrzahl von Punkten (74) an der äußeren oberen Oberfläche (80) des Schuhs leiten.

17. Schuh nach Anspruch 16, wobei der Schuh eine Zunge umfaßt und wenigstens einige der Fasern (72, 74) im freien Ende der Zunge enden.

18. Schuh nach einem der Ansprüche 10, 11 und 16,
dadurch gekennzeichnet,
daß die Punkte dazu eingerichtet sind, ein abstraktes oder geometrisches Muster, ein Logo oder ein oder mehrere alphanumerische Zeichen zu bilden.

19. Schuh nach Anspruch 18,
dadurch gekennzeichnet,
daß die Enden der Fasern an den Punkten mit gefärbten, durchscheinenden Farbstoffen oder Färbungen gefärbt sind.

## Revendications

1. Chaussure comprenant une semelle (16), la chaussure étant destinée à émettre de la lumière ou de l'énergie acoustique lors de l'impact de la semelle (16) sur une surface, la semelle (16) comprenant une unité d'émission de lumière ou de son alimentée par batterie, cette unité comprenant un élément (18) de détection d'impact, une batterie (22), un dispositif (26, 34) émetteur de lumière ou de son, caractérisée en ce que l'élément (18) de détection d'impact est formé d'un matériau piézoélectrique polymère, et en ce que l'unité d'émission comporte en outre un circuit (20) connecté au matériau piézoélectrique (18), ce circuit (20) étant déclenché par l'énergie électrique du matériau piézoélectrique (18) à la suite de l'impact de la semelle (16) sur une surface, si bien que le dispositif (26, 34) d'émission de lumière ou de son est excité à partir de la batterie (22) grâce à l'énergie électrique produite par l'impact.

2. Chaussure selon la revendication 1, caractérisée en ce que le circuit (20) comporte un circuit monostable (56) dont la constante de temps détermine la durée d'excitation du dispositif d'émission de lumière ou de son.

3. Chaussure selon la revendication 2, comprenant un dispositif (58, 60, 62) d'ajustement de la constante de temps du circuit monostable (56).

4. Chaussure selon la revendication 3, caractérisée en ce que le dispositif d'ajustement (58, 60, 62) comporte une résistance variable (58).

5. Chaussure selon l'une quelconque des revendications précédentes, comprenant une diode photoémissive (26) comme dispositif (26) d'émission de lumière.

6. Chaussure selon l'une quelconque des revendications précédentes, caractérisée en ce qu'un dispositif (26) d'émission de lumière est placé à l'intérieur de la semelle (16), et elle comprend en outre un dispositif (70, 72, 74, 76) à fibre optique destiné à conduire la lumière émise par le dispositif photoémissif (26) vers une surface externe de la semelle (16).

7. Chaussure selon l'une quelconque des revendications 1 à 5, caractérisée en ce que la semelle (16) a une structure (12) à semelle et talon moulée en une seule pièce avec au moins la batterie (22), le circuit (20) et un dispositif d'émission de lumière (26) placés dans la partie de talon de la structure (12).

8. Chaussure selon la revendication 7, caractérisée en ce que le dispositif (26) d'émission de lumière est destiné à émettre de la lumière vers l'arrière de la partie de talon (24).

9. Chaussure selon la revendication 7 ou 8, caractérisée en ce que le matériau piézoélectrique polymère (18) est moulé dans la partie de semelle (16) de la structure (12).

10. Chaussure selon l'une quelconque des revendications 1 à 5, comprenant plusieurs dispositifs (26) d'émission de lumière placés en plusieurs points sur la surface supérieure externe (80) de la chaussure.

11. Chaussure selon l'une quelconque des revendications 1 à 5, comprenant plusieurs dispositifs photoémissifs (26) couplés en plusieurs points à la surface supérieure externe (80) de la chaussure par des fibres optiques respectives (82).

12. Chaussure selon la revendication 10 ou 11, caractérisée en ce que les dispositifs sont excités avec une séquence prédéterminée à la suite d'impacts successifs ou de groupes d'impacts détectés par les éléments (18) de détection d'impact.

13. Chaussure selon la revendication 10 ou 11, comprenant plusieurs éléments (18) de détection d'impact répartis dans la semelle (16) de la chaussure, le circuit (20) étant destiné à exciter les dispositifs (26) d'émission de lumière suivant la contrainte relative détectée par chaque élément de détection d'impact (18) afin qu'une indication de la distribution du poids sur la semelle (16) de la chaussure soit donnée.

14. Chaussure selon la revendication 10, caractérisée en ce que le dispositif (70) à fibre optique comporte une seule fibre optique (76) dont une partie au moins est disposée autour de la surface supérieure externe (80) de la chaussure, cette partie ayant plusieurs encoches (78) destinées à émettre de la lumière au niveau de chaque encoche.

15. Chaussure selon la revendication 14, caractérisée en ce que les encoches (78) sont colorées par des colorants ou encres translucides colorées afin que la lumière émise ait une couleur correspondante.

16. Chaussure selon la revendication 6, dans laquelle le dispositif (70) à fibre optique comprend un faisceau de fibres optiques (72, 74), les fibres conduisant la lumière émise en plusieurs points (74) à la surface supérieure externe (80) de la chaussure.

17. Chaussure selon la revendication 16, comprenant une languette, et certaines au moins des fibres (72, 74) se terminent à l'extrémité libre de la languette.

18. Chaussure selon l'une quelconque des revendications 10, 11 et 16, caractérisée en ce que lesdits points sont destinés à former un dessin géométrique ou abstrait, un logo ou un ou plusieurs caractères alphanumériques.

19. Chaussure selon la revendication 18, caractérisée en ce que les extrémités des fibres auxdits points sont colorées par des colorants ou encres translucides colorées.
